(19) Europäisches Patentamt
European Patent Office
Office européen des brevets

(11) **EP 3 277 352 B1**

(12) **EUROPEAN PATENT SPECIFICATION**

(45) Date of publication and mention
of the grant of the patent:
**19.10.2022 Bulletin 2022/42**

(51) International Patent Classification (IPC):
**B29L 31/48** *(2006.01)*    **B29L 31/00** *(2006.01)*
**B33Y 80/00** *(2015.01)*    **A61M 16/06** *(2006.01)*

(21) Application number: **16771097.9**

(52) Cooperative Patent Classification (CPC):
**A61M 16/0605; A61M 16/0666; B33Y 80/00;**
A61M 2207/00; A61M 2210/0618; B29L 2031/4835;
B29L 2031/753

(22) Date of filing: **30.03.2016**

(86) International application number:
**PCT/AU2016/050240**

(87) International publication number:
**WO 2016/154676 (06.10.2016 Gazette 2016/40)**

(54) **IMPROVED SLEEP APNOEA MASK ADAPTER**

VERBESSERTE SCHLAFAPNOEADAPTERMASKE

ADAPTATEUR DE MASQUE CONTRE L'APNÉE DU SOMMEIL AMÉLIORÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.03.2015   AU 2015901138
31.07.2015   AU 2015903049
16.12.2015   AU 2015905214**

(43) Date of publication of application:
**07.02.2018 Bulletin 2018/06**

(73) Proprietor: **Bespoke Medical Innovations Pty Ltd.
Baulkham Hills, New South Wales 2153 (AU)**

(72) Inventor: **HILTON, John
Baulkham Hills, New South Wales 2153 (AU)**

(74) Representative: **Bals & Vogel Patentanwälte
PartGmbB
Konrad-Zuse-Str. 4
44801 Bochum (DE)**

(56) References cited:
**WO-A1-2009/139647      WO-A2-2007/008725
WO-A2-2007/008725      WO-A2-2014/155329
WO-A2-2014/155329      US-A- 5 807 100
US-A1- 2006 023 228      US-A1- 2008 078 396**

## Description

## Technical Field

The invention relates to the field of sleep apnoea equipment. In particular, the invention relates to a device that allows better adherence of a sleep apnoea mask to the face of a person.

## Background of the Invention

Sleep apnoea is commonly treated with equipment providing continuous positive airway pressure (CPAP), typically between 4 and 20 cm $H_2O$ air pressure, to the nasal passage of the patient via a mask that seals around at least the patient's nose.

There are two basic types of CPAP machines (air pumps). Fixed CPAP machines provide a constant and programmable air pressure. Auto-adjusting CPAP machines monitor the airflow and adjust the pressure depending on the detection of various parameters and may provide one pressure for inhalation and a lower pressure during exhalation. The machines typically record the air pressures during use for both indicating the quality of the previous night's therapy for the patient and for use by physicians to review the effectiveness of the therapy over time.

Air is replenished by a continuous air flow through a diffuser that is typically integrated into the mask. The resistance of the diffuser determines the air flow rate that will vary with the delivered air pressure.

These machines measure and can maintain the specified pressure provided there are no excessive air leaks. Since the air supply tube that runs from the machine to the mask, typically 22mm diameter, has very small flow resistance this pressure will usually be the same at the patient's face. The airflow out of the diffuser is therefore constant while the pressure is constant. As the patient breathes in, air for the lungs plus air for the diffuser is drawn through the supply tube; and as the patient breathes out, air from the lungs minus the diffuser air is blown back into the supply tube. This is different from a ventilator which only delivers air from a supply tube.

Although there are a myriad range of masks available on the market there are generally three main types of masks; nasal pillow masks, nasal masks and full-face masks. Most people don't breathe through their mouth when asleep but those that do either need a chin strap to keep their mouth shut or a full-face mask. Nasal pillow masks generally have two bellows, one for each nostril, that sit against the each nostril. Document US 2006/0023228 A1 discloses a process for fabricating a facial mask to custom fit a patient's face for a comfortable fit for facilitating various medical procedures including the steps of generating a 3D data set to define a portion of a patient's face to be fitted with a custom mask, fabricating a patient's mask utilizing a patient's 3D facial data set, and fitting a patient with a custom fit facial mask for facilitating a desired medical procedure.

Nasal masks seal over the bridge of the nose, on the cheek on each side of the nose and on the upper lip underneath the nose. Sometimes, when the supply tube is pulled in certain ways, the soft mask flaps can leak air and generate loud sounds which can disrupt the sleep of the patient or of their partner. Full face masks seal over the mouth and may also seal over the forehead these can be uncomfortable and ungainly.

Current nose-pieces usually have a soft skin contact surface, typically made from soft silicone, to provide as comfortable as possible a fit. Even custom masks made from impressions of a patient's face are usually far more rigid than a generic mask with a flexible nose piece but they still have a relatively soft skin contact surface. The mask straps need to be tight enough to not only oppose the air pressure force but also to keep the mask in place during the normal tugs of the air supply hose and contact with bedding.

For all mask types the straps need to be tightened so the inflated silicone bellows or cushion and the nostrils or face conform to each other to provide an airtight seal. A patient determines their strap tightness by trading off comfort with robustness. Comfort is improved with looser straps while robustness to dislodging forces is improved with tighter straps.

Some air leakage is quite acceptable from a therapy point of view provided it doesn't cause a drop in the supplied air pressure. But air leakage can dry out the skin, eyes and nose, can generate noise and can annoyingly blow on the sleeping partner. It is possible to use humidifier attachments to humidify the air and prevent unwanted dryness of the skin or nose.

US Patent Application No. US 20061023228 A1 discloses a contoured patent interface for use with CPAP treatments and systems wherein the facial seal is formed of flexible and deformable materials.

Accordingly, it is an object of the invention to provide a sleep apnoea mask device that ameliorates at least some of these problems associated with the prior art.

## Summary of the Invention

The invention is defined by the appended claims. In the disclosure is provided a nasal adapter for a sleep apnoea mask that is adapted to receive pressurised air from an existing pressurised air-line via a flexible nose-piece, and is adapted to engage with at least part of a patient's nose including the nostrils; and is adapted to engage with the nose-engaging portion of said nose-piece; and wherein said adapter is constructed from a relatively rigid material. Preferably, the adaptation to engage with said relatively flexible nose-piece has a contact surface shape that allows for considerable relative movement of the rigid mask body while maintaining an airtight seal and also allowing the nasal adapter to generally remain in place on the face.

Preferably, the nose-engaging side of the adapter is personalised to the patient's nose thereby providing a close-fitting engagement.

The invention provides a way for the advantages of the relatively hard nose-engaging surface piece to be combined with the relative flexibility of the softer, more pliable nose-piece. Hard nose-engaging fittings can be subject to being pulled subtly out of place by the movement of the wearer, so the combination with a flexible nose-piece assists with this by resiliently absorbing the minor movements of the patient.

By contrast, subtle movement of a traditional pliable nose-piece can result in leakage of the air around the side of the nose, which can be a particularly annoying to patients as this will direct pressurised air into the patient's eyes. In this way, the use of the relatively rigid adapter can prevent these type of leakage problems by providing a more reliable seal.

Whereas, the rigid nose adapter provided by the invention helps to provide a more robust fit to withstand these common dislodging forces, added to the flexibility provided by the pliable nose-piece.

The features of the nasal adapter that interface to the nasal nose-piece, or mask, are designed to employ and optimise the moveable air cushion effect the original mask designers intended for the flexible silicone portion. The designers sought to maintain a mostly airtight seal as the rigid portion of the mask, to which both the flexible silicone portion and the air supply tube are affixed and which often includes a diffuser, is moved around due to dislodging tugs on the air supply tube and dislodging forces due to mask and strap contact and movement against nearby items such as bedding. Many nasal masks employ a pliable silicone skirt which is intended to sit on the skin and generally not slide as the rigid mask platform moves around but rather to have a rolling type of motion where the portion of the skirt that is held off the skin due mainly to air pressure moves laterally in relation to the portion of the nearby skirt that is against the skin.

This type of silicone skirt design is largely successful but there is difficulty providing a robust seal on either side of the nose bridge due to the geometry of this area leading to very little air sealing force. The result is that many users suffer from air blowing towards and tending to dry out their eyes. Notably, the nasal adapter is shaped to ensure a more robust seal in this area.

The combination of the present adapter with an existing nasal mask is a device with a relatively rigid skin contacting member in contact with or connected to a flexible bellows-like member that is also in contact with or connected to a relatively rigid main mask body to which the straps and air line are attached. The present invention allows the relatively rigid platform to suffer increased dislodgment from a central position, due to dislodging forces from the air supply tube or from bedding, without a troublesome air leak occurring compared to existing sleep apnoea masks.

Changes in air pressure result in a change in the average contact pressure of the nasal adapter on the face. A sealing coefficient is defined as the ratio of the change in average contact pressure to the change in air pressure minus one and is typically expressed as a percentage. The average contact pressure of a mask and nasal adapter having a positive sealing coefficient will increase and decrease faster than the corresponding change in air pressure. This means the mask and nasal adapter inherently tend to seal better as air pressure increases. For example, increasing the air pressure by 10% may produce a 15% increase in skin contact pressure. A design having a negative sealing coefficient will inherently leak air once the air pressure reaches the contact pressure and starts pushing the nasal adapter away from the face.

A positive sealing coefficient results in a better seal being achieved as the sealing force on the nasal adapter arising solely from the pressurized air can be relied upon to provide an adequate seal. Any other sealing forces, such as those arising from stresses in the side walls of the silicone cushion, are unnecessary and thereby allow for nasal adapter and mask designs that maintain a good seal over a greater range of movement of the mask body compared with existing mask designs. This in turn allows for lighter strap forces as the straps may be left loose before the air is pressurized, the pressurized air then inflating the silicone cushion which pushes the mask body and the nasal adapter apart until the straps tighten to oppose the force of the pressurized air on the mask body.

Note that existing mask designs have a small positive sealing coefficient due to the contact cross sectional area being slightly smaller than the pressurized air cross sectional area. The physical characteristics of a soft cushion only allow for designs within a very small range of sealing coefficients. The physical characteristics of a rigid nasal adapter allow for designs within a considerable range of sealing coefficient.

For a given nasal mask a nasal adapter can be designed with an appropriate skin contact area to obtain a far superior balance between comfort and robustness compared existing CPAP masks. Designs having sealing coefficients of about 20% have produced excellent results.

According to another aspect of the invention, the nasal adapter is adapted to receive pressurized air from an existing pressurized air-line, and is adapted to engage with at least part of a patient's nose including the nostrils via a soft cushion, wherein said adapter is constructed from a relatively rigid material, and wherein the adapter is constructed such that the largest cross-sectional area of said soft cushion that engages with the pressurized air-line, and the area of the skin contact region of said adapter engaged with said patient's face have relative sizes such that a positive sealing coefficient for the mask and nasal adapter is achieved.

Preferably, the cross sectional area of the largest pressurised air cross section within the soft cushion ($A_A$) minus the sum of the cross sectional areas defined by the internal edges of the skin contact region around the patient's nostrils ($A_N$) is greater than the cross sectional area of the outer edge of the skin contact region (As) minus the sum of the cross sectional areas defined by the internal edges of the skin contact region around the patient's nostrils ($A_N$); said cross sections being defined as being normal to the axis along which the sealing force acts to press the nasal adapter onto the patient's face.

The nasal adapter fits between the silicone cushion of a CPAP mask and the skin. The forces acting on the nasal adapter, being a relatively rigid item, are due to contact with the pressurized air, the skin, the silicone cushion and ambient air plus the weight of the nasal adapter. Measuring pressurized air pressure and skin contact pressures relative to ambient air pressure simplifies calculations as this eliminates ambient air pressure from the mathematical formulae. For practical purposes the effect of gravity is negligible and can be ignored.

Nasal pillow CPAP masks have two nostril prongs, each prong engaging with a nostril, the prongs having a short hollow 'trunk' region that connects the nostril engaging prong with the main part of the nasal pillow silicone cushion. The prongs are designed to provide a high degree of movement of the nostril engaging prong in relation to the main cushion and so to provide as robust a seal as possible as the mask body experiences dislodging forces. One form of the invention has two flanges, each flange having a groove for receiving a cut-off trunk. The nasal pillow cushion is modified by cutting both trunks to remove the nostril engaging prong and each cut-off trunk stretched over the corresponding flange and snugly fitting into the groove to provide an air-tight seal.

This nasal pillow type of adapter does not rely on internal air pressure but rather requires the head straps to be tightened appropriately to provide the skin contact sealing pressure.

According to another embodiment of the invention, there is provided the use of a nasal adaptor, as per those described above, to produce a better fit between a sleep apnoea mask and a patient's skin.

According to another embodiment of the invention, there is provided a sleep apnoea mask that incorporates the properties of the nasal adaptor defined above.

Now will be described, by way of a specific, non-limiting example, a preferred embodiment of the invention with reference to the drawings.

## Brief Description of the Drawings

Figure 1 is a diagram of a mask adapter according to the invention, shown from the CPAP mask engaging side.

Figure 2 is a diagram of the adapter of figure 1 shown from the nose-engaging side.

Figure 3 is a diagram of an alternative mask adapter according to the invention, shown from the CPAP mask engaging side.

Figure 4 is a diagram of the adapter of figure 3 shown from the nose-engaging side.

Figure 5 is a diagram of the adapter according of figure 1 fitted to a CPAP nasal mask.

Figure 6 is a frontal schematic view illustrating the cross sectional areas of a mask adapter according to the invention.

Figure 7 is a schematic side view that illustrates the overall sealing force applied to a nasal adapter according to the invention.

Figure 8 is a schematic graph illustrating the general relationship between air pressure at the skin and in the mask at different sealing coefficients.

## Detailed Description of the invention

One embodiment of the disclosure resides in a relatively rigid adapter that may be applied to a sleep apnoea treatment apparatus, said apparatus comprising a CPAP machine, an attached air delivery line and a nasal mask adapted to deliver the air to a patient's nostrils, in the form of a nasal mask adaptor that delivers air from the air-line to the patient's nostrils in a more comfortable an efficient manner that known in the prior art.

The adapter is shaped to fit snugly to the patient's nose on one side, and adapted to fit on to the pliable silicone nose-piece or mask of existing CPAP equipment, such as those produced by Fisher & Paykel Healthcare, ResMed and Philips Respironics.

In a preferred embodiment the nasal mask interface geometry is shaped so as to maintain an airtight seal over as wide a range of movement as possible of the mask body in relation to the adapter and so provide a robust seal as dislodging forces act on the mask body. A suitable retaining lip may be provided at the opening of the nasal mask interface to conveniently prevent the adapter from falling out of the mask whenever the mask is taken off the face. During night-time movement the silicone skirt typically does not slide on the adapter but rather tends to freely roll over the surface.

In other preferred embodiments the relatively rigid skin contact portion is connected to the rigid mask platform with a flexible tube-like member securely attached to both the rigid skin contact portion and the rigid mask platform. The rigid mask platform can move around due to dislodging forces from the straps and the air supply tube while the air pressure and possibly the spring-like nature of the flexible tube-like member keep the relatively rigid skin contact member in place on the patient's face.

In other embodiments a spring-like member may be used to keep the relatively rigid skin contact member in place on the patient's face.

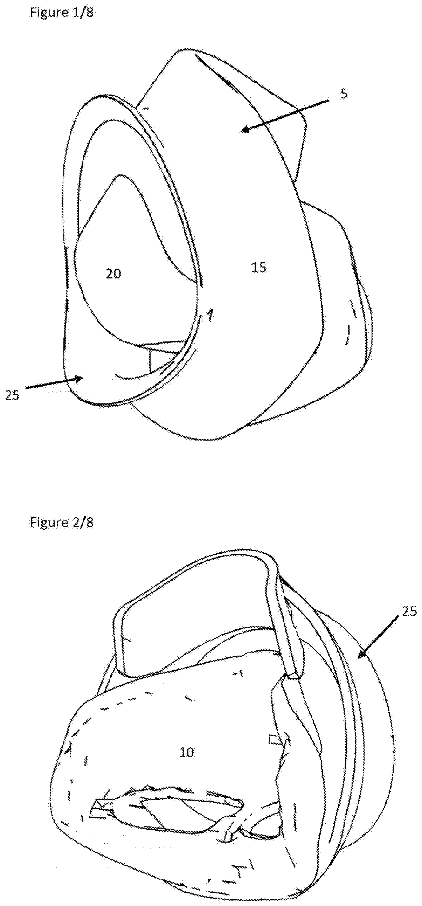

Turning to figures 1 and 2, there is shown a nasal adaptor according to the invention, from various angles.

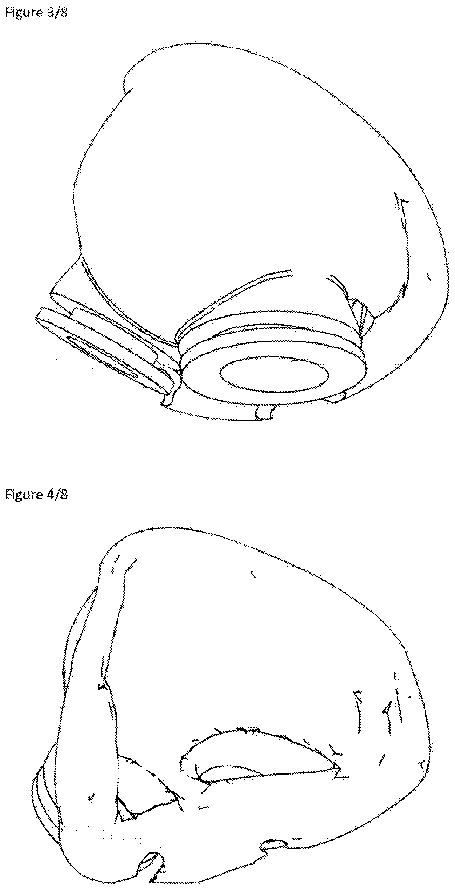

Figures 3 and 4 show similar views of an alternative design for the nasal adapter that is adapted to fit to a differently configured CPAP mask.

The nasal adapter 5 is constructed from a hard, bio-compatible polymer such as acrylonitrile butadiene styrene (ABS) plastic, polycarbonate plastic, polyurethane, poly lactic acid (PLA) plastic and polyethylene plastic. It attaches to the mask body on the air supply side, and has a surface 10 adapted to the exact contour of the patient's nose and nostrils on the patient side, as illustrated in figure 2. This contour is achieved by digitising the surface of the patient's nose and creating an exact match for their nose by e.g. 30 printing.

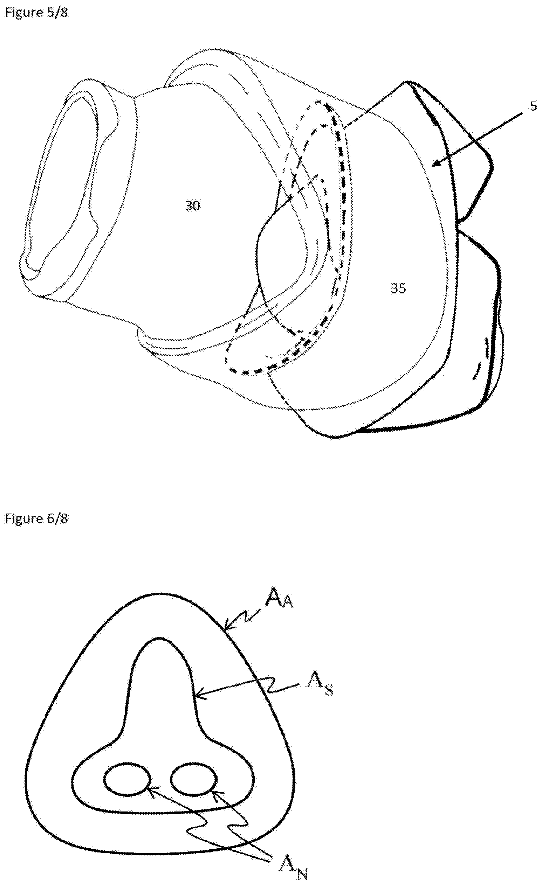

This embodiment of the adapter further features a continuous 'wall' 15 that extends from the machine side surface 20. The profile of the wall 15 is in the shape of a 'rounded triangle' and it is shaped to fit sealingly inside the CPAP nasal mask shown on figure 5.

The wall 15 also features a lip 25 at the periphery to assist in retention of the adapter in the pliable nasal mask when the mask is removed from the face.

Figure 5 shows the adapter 5 fitted in to a pliable silicone nasal mask 35. The patient-side contour may be achieved by using a digitising process to capture the exact contours of the patient's face, which is then used to create the mask via a 30 printing process.

In engineering the concept of a free body diagram is commonly used to analyse forces on a rigid body. The forces on the main mask body are due to the straps, the pressurized air, the skin contact pressure and the tugs from the air supply tube. Contact of the mask with a pillow or bedding is also a consideration. Comfort is increased by minimizing the maximum skin contact pressure by providing an even and light pressure over a large skin area. The 'ala', i.e. the outer soft rounded portion of the nostril, will itself tend to bellow out into a rigid nose adapter that seals around the nostril openings.

Nasal pillow masks typically have a strap either side of the mask that each pull up somewhere over the ear. The plane formed by the two strap forces needs to be positioned to deliver a retaining force resulting in as even as possible a pressure where the mask and skin form an airtight seal. Masks with three or more straps, or even with a rigid member extending to contact the forehead, provide a more robust placement but with greater obstruction of the area in front of the face.

The mask's internal air pressure exerts an ejection force onto the adapter. This force can be approximated by multiplying the cross-section of the air cavity in the silicone cushion that is normal to the ejection force direction by the air pressure. The larger the cross-section of the pressurised air, the greater the ejection force and therefore the greater the strap force needed to oppose it. The minimum pressurized cross section of a theoretically ideal mask is equal to the nostril opening cross section.

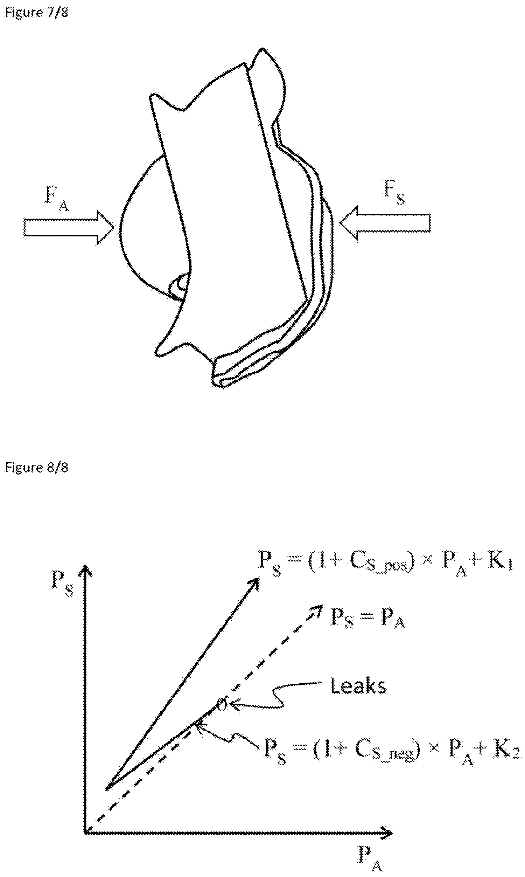

Turning to figures 6 and 7, one can see frontal and side views of an adapter according to the invention that illustrates the overall sealing force, $F_A$, applied to the nasal adapter by both the silicone cushion and the pressurized air and the equal and opposite opposing force, Fs, resulting from both skin contact pressures and any pressurized air acting on the face side of the nasal adapter.

The instantaneous sealing coefficient is defined as the rate of change of the average skin contact pressure to the rate of change of the pressurized air pressure minus one. The formula is

$$C_S = (\Delta P_S / \Delta P_A) - 1$$

where

Cs is the sealing coefficient
Ps is the average skin contact pressure
$P_A$ is the pressurized air pressure

A positive sealing coefficient has the average skin pressure increasing and decreasing faster than changes to the pressurized air pressure. Conversely a negative sealing coefficient has the average skin pressure increasing and decreasing more slowly. The sealing coefficient is generally fairly constant over the pressure ranges used with CPAP therapy. The use of a constant sealing coefficient simplifies the following explanation. A constant sealing coefficient, although common, is not necessary for the present invention as the same principles would still apply.

The average skin contact pressure can be written as a function of the air pressure as

$$P_S = (1 + C_S) \times P_A + K$$

where K is a constant.

To provide an airtight seal the skin contact pressure just adjacent to the boundary where the skin, the nasal adapter and the pressurized air meet must be greater than the pressurized air pressure otherwise the air will push the skin aside until it leaks out. A nasal adapter with a negative sealing coefficient will eventually leak at some point as the pressurized air pressure is increased while a nasal adapter with a positive sealing coefficient will not, as in the latter case the force applied by the inflation of the mask tends to push the adapter toward the face and as that pressure (and therefore the force) increases, its effect is to seal the adapter ever-tighter on to the face. This is illustrated by the graph shown in Figure 8.

Skin contact pressures are very difficult to measure. It is more convenient and practical to use cross sectional areas to estimate the sealing coefficient. Figure 6 shows cross sectional areas that are normal to the axis along which the forces $F_A$ and $F_S$ act. The cross sectional area specifying the area measurement $A_A$ is the largest cross section of the pressurized air cavity. The cross sectional area specifying the area measurement $A_S$ is that of the outside of the skin contact region. The sum of the cross sectional areas defined by the internal edges of the skin contact region specify the area measurement $A_N$.

The sealing and reaction forces, $F_A$ and $F_S$, are a function of pressurized air pressure, $P_A$, average skin contact pressure, $P_S$, and the relevant cross sectional areas.

$$F_A = P_A \times (A_A - A_N) \quad F_S = P_S \times (A_S - A_N)$$

For equilibrium

$$F_S = F_A$$

So, for changes in pressurized air pressure

$$\Delta P_S \times (A_S - A_N) = \Delta P_A \times (A_A - A_N)$$

hence

$$\Delta P_S \,/\, \Delta P_A = (A_A - A_N) \,/\, (A_S - A_N)$$

Substituting to make $C_S$ a function of the three cross sectional areas;

$$C_S = (\Delta P_S \,/\, \Delta P_A) - 1$$

$$C_{S\_AREA} = ((A_A - A_N) \,/\, (A_S - A_N)) - 1$$

where $C_{S\_AREA}$ is an approximation of $C_S$.

Figure 8 is a graph representing a plot of pressure force applied at the patient's skin as the pressure supplied by the CPAP machine is increased, for different theoretical adapter designs.

In plot 1, the force increase at the patient's nose is equal to the force applied by increase in pressure from the CPAP machine. This represents a 'neutral' sealing coefficient of zero.

In plot 2, the force at the patient's nose rises less sharply than the force applied by the pressurized air. It begins above plot 2 by virtue of e.g. the additional force applied by the mask straps. However, as the air pressure is increased the likelihood of leakage increases, and the point at which plot 2 intersects with plot 1, the air pressure force applied by the CPAP machine has overcome the mask's ability to seal to the patient's face, and so leakage will occur.

In plot 3, the force at the patient's nose rises more sharply than the force applied by the pressurized air from the CPAP machine. So the plot always remains above plot 1, meaning that within practical bounds, the mask will be unlikely to leak because the increase in CPAP pressure will simply cause the adapter to adhere more robustly to the patient's face.

The practical upshot of this phenomenon is that, because force is a function of area times pressure, it has been found that where the cross sectional surface area of the part of the adapter that contacts the patient's face is smaller than the cross sectional surface area that is in contact with either the pressurized air or the silicone cushion the greater the increase in average skin contact pressure for an increase in air pressure. This makes it harder for the mask to be dislodged by random movements of the patient during sleep, and makes for a more comfortable overall experience for the patient.

It also means that a relatively small adapter can be designed that still adheres well to the face.

For example, it has been observed that where the contact area on the face of the adapter is about 20% smaller than the contact area with the mask on the of the air supply line side of the adapter, the balance of forces in the system resolve to a net force pushing the adapted toward the patient's face.

One process for manufacturing a preferred embodiment involves digitizing the patient's nose and surrounding face to create a 30 computer surface. This surface is used to create a 30 model of the custom nose adapter including any features to attach it to the mask.

Preferably, the adaptor is made from a material selected from the group comprising: Acrylonitrile butadiene styrene (ABS) plastic, polycarbonate plastic, polyurethane, poly lactic acid (PLA) plastic and polyethylene plastic. Other bio-compatible materials may also be used.

As stated above, the adaptor may be made from a rigid material selected from the group comprising: Acrylonitrile butadiene styrene (ABS) plastic, polycarbonate plastic, polyurethane, poly lactic acid (PLA) plastic and polyethylene plastic. The foam pad on the nose bridge is made from a low to moderate durometer material such as neoprene and polyolefin. Other bio-compatible materials may also be used.

Advantageously, the nasal adaptor may be produced by a combination of digitisation of the patent's nose and fabrication via a process of 30 printing followed by surface preparation to provide a smooth and hygienic surface. This technique provides a very closely fitting and comfortable rigid nasal adaptor, due to the accuracy and precision of the digitisation and 30 printing process, and which tends not to be susceptible to bacterial or other fouling due to the hygienic surface finish.

A 3O-printed material may have tiny air-filled voids and some surface porosity that can harbour bacteria. In a preferred embodiment the 30 printed part is coated in a biocompatible and bacterially resistant material, for example polyurethane. The coating could be sprayed, brushed, dipped or otherwise applied to the 30 printed part. In another embodiment the 30 printed part is dipped in a liquid solvent or bathed in a solvent gas to partially liquefy and reform the exterior surface to form a hygienic and non-porous surface finish.

Even though there are benefits in a rigid nose adapter there may be a market preference for a semi-rigid or soft nose adapter.

In other embodiments the nose adapter includes the air diffuser. In one such embodiment a second wall with small holes forming the air diffuser is offset from the wall that is in contact with the outside of the nose. This provides a larger diffuser area than is common in existing masks and so will have slower air movement for the same airflow rate.

In other embodiments the nose adapter includes the air diffuser. In one such embodiment a second wall with small holes forming the air diffuser is offset from the wall that is in contact with the outside of the nose. This provides a larger diffuser area than is common in existing masks and so will have slower air movement for the same airflow rate.

## Claims

1. A nasal adapter (5) and flexible nose-piece (30) for a sleep apnoea mask (1, 2, 3, 4, 30) that is adapted to receive pressurised air from a pressurised air-line, the sleep apnoea mask (1, 2, 3, 4, 30) comprising a mask body, an air supply tube and mask straps to hold the sleep apnoea mask (1, 2, 3, 4, 30) in place on a patient's face during sleep, the nasal adapter (5) being configured to engage with at least part of a patient's nose including the nostrils, and being **characterised by**:

   a mask engaging side (20) configured to be attached to the flexible nose-piece (30) and configured to attach the nasal adapter (5) to the mask body of the sleep apnoea mask (1, 2, 3, 4, 30) via a flexible nose-piece (30) such as a bellows-like or tube-like member, such that the flexible nose-piece (30) is positioned between the nasal adapter (5) and the mask body to thereby allow relative movement between the nasal adapter (5) and the mask body in response to dislodging forces acting on the sleep apnoea mask (1, 2, 3, 4, 30);
   a nose-engaging side (10) comprising a relatively rigid skin contact portion around the patient's nose including the nostrils, wherein the nose-engaging side (10) is personalised to the patient's nose contour and nostrils using a digitising process to capture the exact contours of the patient's nose and nostrils, to thereby provide a close-fitting engagement; and
   the nasal adapter (5) being constructed from a hard, biocompatible polymer to form said relatively rigid skin contact portion.

2. The nasal adapter (5) of claim 1, wherein the mask-engaging side (20) comprises a projection extending from the adapter (5) in the form of a continuous wall (15) shaped to engage with said flexible nose-piece.

3. The nasal adapter (5) of claim 2, wherein said wall (15) is roughly triangular with curved vertices.

4. The nasal adapter (5) of claim 1, wherein the flexible nose-piece comprises a soft cushion and wherein the adapter (5) is constructed such that the largest cross-sectional area of said soft cushion that engages with the pressurized air-line, and the cross-sectional area of the skin contact region of said adapter (5) engaged with said patient's face have relative sizes such that a positive sealing coefficient for the mask (1, 2, 3, 4, 30) is achieved, the cross-sectional area of the skin contact region being normal to the engaging force, said positive sealing coefficient arising where the rate of change of pressure experienced toward the patient's skin is greater than the rate of change of supplied air pressure.

5. The nasal adapter (5) of claim 4, wherein the cross sectional area of the largest pressurised air cross section within the soft cushion ($A_A$) minus the sum of the cross sectional areas defined by the internal edges of the skin contact region around the patient's nostrils ($A_N$) is greater than the cross sectional area of the outer edge of the skin contact region (As) minus the sum of the cross sectional areas defined by the internal edges of the skin contact region around the patient's nostrils ($A_N$); said cross sections being defined as being normal to the axis along which the sealing force acts to press the nasal adapter (5) onto the patient's face.

6. The nasal adapter (5) of claim 5, wherein ($A_A$-$A_N$) is at least 5% larger than (As-AN).

7. The nasal adapter (5) of claim 5, wherein ($A_A$-$A_N$) is at least 10% larger than (As- $A_N$).

8. The nasal adapter (5) of claim 5, wherein ($A_A$-$A_N$) is at least 20% larger than (As- $A_N$).

9. The nasal adapter (5) of claim 5, wherein ($A_A$-$A_N$) is no more than 50% larger than (As-$A_N$).

**10.** The nasal adapter (5) of any preceding claim, wherein the rigid material is a material selected from the group comprising: acrylonitrile butadiene styrene (ABS) plastic, polycarbonate plastic, polyurethane, polylactic acid (PLA) plastic and polyethylene plastic.

**11.** The nasal adapter (5) of claim 1, wherein said nasal adapter (5) has been manufactured by a process of printing.

**12.** The nasal adapter (5) of claim 1, wherein said adapter (5) is coated in a biocompatible and bacterially resistant material.

**13.** The nasal adapter (5) of claim 12, wherein said adapter (5) is coated in polyurethane.

**14.** The nasal adapter (5) of claim 1, further comprising a pad for contact with the patient's nose bridge, the pad being made from a low to moderate durometer material.

**15.** The nasal adapter (5) of claim 1, wherein the mask (1, 2, 3, 4, 30) engaging side is configured to securely attach to the nasal patient interface.


**Patentansprüche**

**1.** Nasenadapter (5) und flexibles Nasenteil (30) für eine Schlafapnoemaske (1, 2, 3, 4, 30) die ausgelegt ist, um Druckluft von einer Druckluftleitung zu empfangen, wobei die Schlafapnoemaske (1, 2, 3, 4, 30) einen Maskenkörper, einen Luftzufuhrschlauch und Maskenbänder zum Halten der Schlafapnoemaske (1, 2, 3, 4, 30) während des Schlafs auf dem Gesicht eines Patienten aufweist, wobei der Nasenadapter (5) so konfiguriert ist, dass er mit mindestens einem Teil der Nase eines Patienten, insbesondere der Nasenlöcher, in Eingriff kommt, und **gekennzeichnet ist durch**:

eine Maskeneingriffsseite (20), die so konfiguriert ist, dass sie an dem flexiblen Nasenteil (30) angebracht werden kann und so konfiguriert ist, dass sie den Nasenadapter (5) an dem Maskenkörper der Schlafapnoemaske anbringt (1, 2, 3, 4, 30) über ein flexibles Nasenstück (30), wie z. B. ein balg- oder schlauchartiges Element, so dass das Flexible Nasenstück (30) zwischen dem Nasenadapter (5) und dem Maskenkörper positioniert ist, um dadurch eine relative Bewegung zwischen dem Nasenadapter (5) und dem Maskenkörper als Reaktion auf auf die Schlafapnoemaske (1, 2, 3, 4, 30) wirkende Verschiebungskräfte zu ermöglichen (1, 2, 3, 4, 30); eine Naseneingriffsseite (10), die einen relativ starren Hautkontaktabschnitt um die Nase des Patienten insbesondere der Nasenlöcher umfasst, wobei die Naseneingriffsseite (10) unter Verwendung eines Digitalisierungsverfahrens an die Nasenkontur und die Nasenlöcher des Patienten angepasst wird, um die exakten Konturen der Nase und der Nasenlöcher des Patienten zu erfassen und dadurch einen eng anliegenden Eingriff zu schaffen; und wobei der Nasenadapter (5) aus einem harten, biokompatiblen Polymer hergestellt ist, um den relativ starren Hautkontaktabschnitt zu bilden.

**2.** Der Nasenadapter (5) nach Anspruch 1, wobei die Maskeneingriffsseite (20) einen Vorsprung umfasst, der sich vom Nasenadapter (5) in Form einer durchgehenden Wand (15) erstreckt, die so geformt ist, dass sie mit dem flexiblen Nasenteil in Eingriff kommt.

**3.** Der Nasenadapter (5) nach Anspruch 2, wobei die Wand (15) ungefähr dreieckig mit gekrümmten Spitzen ist.

**4.** Nasenadapter (5) nach Anspruch 1, wobei das flexible Nasenteil ein weiches Kissen umfasst und wobei der Nasenadapter (5) so konstruiert ist, dass die größte Querschnittsfläche des weichen Kissens, die mit der Druckluftleitung in Eingriff steht, und die Querschnittsfläche des Hautkontaktbereichs des Nasenadapters (5), der mit dem Gesicht des Patienten in Eingriff steht, relative Größen aufweisen, so dass ein positiver Dichtungskoeffizient für die Maske (1, 2, 3, 4, 30) erreicht wird, wobei die Querschnittsfläche des Hautkontaktbereichs normal zur Eingriffskraft ist, wobei der positive Dichtungskoeffizient entsteht, wenn die Änderungsrate des Drucks, der auf die Haut des Patienten wirkt, größer ist als die Änderungsrate des zugeführten Luftdrucks.

**5.** Nasenadapter (5) nach Anspruch 4, wobei die Querschnittsfläche des größten Druckluftquerschnitts innerhalb des weichen Kissens ($A_A$) abzüglich der Summe der Querschnittsflächen, die durch die Innenkanten des Hautkontaktbereichs um die Nasenlöcher ($A_N$) des Patienten definiert sind, größer ist als die Querschnittsfläche der Außenkante des Hautkontaktbereichs (As) abzüglich der Summe der Querschnittsflächen, die durch die Innenkanten des Hautkontaktbereichs um die Nasenlöcher ($A_N$) des Patienten definiert sind; wobei die Querschnitte so definiert sind, dass sie senkrecht zu der Achse verlaufen, entlang der die Dichtkraft wirkt, um den Nasenadapter (5) auf das Gesicht des Patienten zu drücken.

**6.** Der Nasenadapter (6) nach Anspruch r, wobei (A$_A$-A$_N$) mindestens 5 % größer ist als (As- A$_N$).

7. Der Nasenadapter (5) nach Anspruch 5, wobei (A$_A$-A$_N$) mindestens 10 % größer ist als (A$_S$- A$_N$).

8. Der Nasenadapter (5) nach Anspruch 5, wobei (A$_A$-A$_N$) mindestens 20 % größer ist als (A$_S$- A$_N$).

9. Der Nasonadapter (5) nach Anspruch 5, wobei (A$_A$-A$_N$) nicht mehr als 50 % größer ist als (As- A$_N$).

10. Der Nasenadapter (5) nach einem der vorhergehenden Ansprüche, wobei das starre Material ein Material ist, das aus der Gruppe ausgewählt ist, die umfasst: Acrylnitril-Butadien-Styrol (ABS)-Kunststoff, Polycarbonat-Kunststoff, Polyurethan, Polymilclisäure (PLA)-Kunststoff und Polyethylen-Kunststoff.

11. Der Nasenadapter (5) nach Anspruch 1, wobei der Nasenadapter (5) durch ein Druckverfahren hergestellt wurde.

12. Der Nasenadapter (5) nach Anspruch 1, wobei der Adapter (5) mit einembiokompatiblen und bakterienresistenten Material beschichtet ist.

13. Der Nasenadapter (5) nach Anspruch 12, wobei der Adapter (5) mit Polyurethan beschichtet ist.

14. Der Nasenadapter (5) nach Anspruch 1 umfassend ferner ein Polster für den Kontakt mit dem Nasenrücken des Patienten, wobei das Polster aus einem Material mit niedriger bis mittlerer Härte hergestellt ist.

15. Der Nasenadapter (5) nach Anspruch 1, wobei die Maskeneingriffsseite (1, 2, 3, 4, 30) so konfiguriert ist, dass sie sicher an der nasalen Patientenschnittstelle befestigt werden kann.

**Revendications**

1. Adaptateur nasal (5) et pièce nasale flexible (30) pour un masque d'apnée du sommeil (1, 2, 3, 4, 30) qui est adapté pour recevoir de l'air sous pression d'une conduite d'air sous pression, le masque d'apnée du sommeil (1, 2, 3, 4, 30) comprenant un corps de masque, un tube d'alimentation en air et des sangles de masque pour maintenir le masque d'apnée du sommeil (1, 2, 3, 4, 30) en place sur le visage d'un patient pendant le sommeil, l'adaptateur nasal (5) étant configuré pour s'engager avec au moins une partie du nez d'un patient, en particulier les narines, et étant **caractérisé par** :

un côté d'engagement du masque (20) configuré pour être fixé au pièce nasale flexible (30) et configuré pour fixer l'adaptateur nasal (5) au corps du masque d'apnée du sommeil (1, 2, 3, 4, 30) par l'intermédiaire d'un pièce nasal flexible (30) tel qu'un élément en forme de soufflet ou de tube, de sorte que le pièce nasal flexible (30) est positionné entre l'adaptateur nasal (5) et le corps du masque pour permettre ainsi un mouvement relatif entre l'adaptateur nasal (5) et le corps du masque en réponse aux forces de délogement agissant sur le masque d'apnée du sommeil (1, 2, 3, 4, 30);

un côté de mise en prise avec le nez (10) comprenant une partie de contact avec la peau relativement rigide autour du nez du patient, en particulier les narines, dans lequel le côté de mise en prise avec le nez (10) est personnalisé en fonction du contour du nez et des narines du patient en utilisant un processus de numérisation pour capturer les contours exacts du nez et des narines du patient, afin de fournir ainsi un engagement à ajustement serré ; et

l'adaptateur nasal (5) étant construit à partir d'un polymère dur et biocompatible pour former ladite partie de contact avec la peau relativement rigide.

2. Adaptateur nasal (5) selon la revendication 1, dans lequel le côté d'engagement du masque (20) comprend une projection s'étendant depuis l'adaptateur nasal (5) sous la forme d'une paroi continue (15) formée pour s'engager avec ladite pièce nasale flexible.

3. Adaptateur nasal (5) selon la revendication 2, dans lequel ladite paroi (15) est grossièrement triangulaire avec des sommets incurvés.

4. Adaptateur nasal (5) selon la revendication 1, dans lequel le pièce nasale flexible comprend un coussin souple et dans lequel l'adaptateur nasal (5) est construit de telle sorte que la plus grande surface de section transversale dudit coussin souple qui s'engage avec la conduite d'air sous pression, et la surface de section transversale de la région de contact avec la peau dudit adaptateur nasal (5) engagée avec le visage dudit patient ont des tailles relatives telles qu'un coefficient d'étarichéité positif pour le masque (1, 2, 3, 4, 30), l'aire de la section transversale de la région de contact avec la peau étant normale à la force d'engagement, ledit coefficient d'étanchéité positif apparaissant lorsque le taux de changement de la pression subie par la peau du patient est supérieur au taux de changement de la pression de l'air fourni.

5. Adaptateur nasal (5) selon la revendication 4, dans lequel la section transversale de la plus grande section transversale d'air sous pression à l'intérieur du coussin mou (A$_A$) moins la somme des sections transversales définies par les bords internes de la région de contact avec la peau autour des narines du patient ($A_N$) est supérieure à la section transversale du bord externe de la région de contact avec la peau (As) moins la somme des sections transversales définies par les bords internes de la région de contact avec la peau autour des narines du patient ($A_N$) ; lesdites sections transversales étant définies comme étant normales à l'axe le long duquel la force d'étanchéité agit pour presser l'adaptateur nasal (5) sur le visage du patient.

6. Adaptateur nasal (5) selon la revendication 5, dans lequel ($A_A$-$A_N$) est au moins 5 % plus grand que ($A_S$-$A_N$).

7. Adaptateur nasal (5) selon la revendication 5, dans lequel ($A_A$-$A_N$) est au moins 10 % plus grand que ($A_S$- $A_N$).

8. Adaptateur nasal (5) selon la revendication 5, dans lequel ($A_A$-$A_N$) est au moins 20 % plus grand que (As- $A_N$).

9. Adaptateur nasal (5) selon la revendication 5, dans lequel ($A_A$-$A_N$) n'est pas plus de 50% plus grand que (As- $A_N$).

10. Adaptateur nasal (5) selon l'une des revendications précédentes, dans lequel le matériau rigide est un matériau choisi dans le groupe comprenant ; le plastique acrylonitrile-butadiène-styrène (ABS), le plastique polycarbonate, le polyuréthane, le plastique acide polylactique (PI.A) et le plastique polyéthylène.

11. Adaptateur nasal (5) selon la revendication 1, dans lequel ledit adaptateur nasal (5) a été fabriqué par un procédé d'impression.

12. Adaptateur nasal (5) selon la revendication 1, dans lequel ledit adaptateur nasal (5) est revêtu en un matériau biocompatible et résistant aux bactéries.

13. Adaptateur nasal (5) selon la revendication 12, dans lequel ledit adaptateur nasal (5) est revêtu de polyuréthane.

14. Adaptateur nasal (5) selon la revendication 1, comprenant en outre un coussinet destiné à entrer en contact avec l'arête nasale du patient, le coussinet étant fait d'un matériau à duromètre faible à modéré.

15. Adaptateur nasal (5) selon la revendication 1, dans lequel le côté d'engagement du masque (1, 2, 3, 4, 30) est configuré pour se fixer solidement à l'interface nasale du patient.

Figure 1/8

Figure 2/8

Figure 3/8

Figure 4/8

Figure 5/8

Figure 6/8

Figure 7/8

Figure 8/8

$$P_S = (1 + C_{S\_pos}) \times P_A + K_1$$

$$P_S = P_A$$

Leaks

$$P_S = (1 + C_{S\_neg}) \times P_A + K_2$$

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060023228 A1
- US 20061023228 A1